(19) Europäisches Patentamt
European Patent Office
Office européen des brevets

(11) Publication number: **0 045 157 B1**

(12) **EUROPEAN PATENT SPECIFICATION**

(45) Date of publication of the patent specification: **18.04.84**

(51) Int. Cl.³: **A 61 K 31/365**, C 07 H 17/08, C 12 P 19/62 // (C12P19/62, C12R1/54)

(21) Application number: **81303232.3**

(22) Date of filing: **14.07.81**

(54) **Macrolide antibiotics, their preparation and veterinary compositions containing them.**

(30) Priority: **24.07.80 GB 8024211**

(43) Date of publication of application: **03.02.82 Bulletin 82/5**

(45) Publication of the grant of the patent: **18.04.84 Bulletin 84/16**

(84) Designated Contracting States: **BE CH DE FR GB IT LI LU NL SE**

(56) References cited:
**US - A - 4 201 843**

**CHEMICAL & PHARMACEUTICAL BULLETIN, vol. 28, no. 6, June 1980, pages 1963-1965, edit. by Pharmaceutical Society of Japan SATOSHI OMURA et al.: "Isolation and characterization of a new 16-membered lactone, protylonolide, from a mutant of tylosin-producing strain, streptomyces fradiae KA-427 1,2"**
**THE JOURNAL OF ANTIBIOTICS, vol. XXX, no. 11, November 1977, pages 1012-1014, edit. by Japan antibiotics res. association J.W. CORCORAN: "Relationship of of ribosomal binding and antibacterial properties of tylosin-type antibiotics"**

(73) Proprietor: **ELI LILLY AND COMPANY, 307, East McCarty Street, Indianapolis Indiana 46285 (US)**

(72) Inventor: **Baltz, Richard Henry, 7446 Sunset Lane, Indianapolis Indiana (US)**
Inventor: **Wild, Gene Muriel, 7455 Jewel Lane, Indianapolis Indiana (US)**
Inventor: **Seno, Eugene Thomas, Cottage No. 1 John Innes Institute, Colney Lane Norwich NR4 7UH (GB)**

(74) Representative: **Hudson, Christopher Mark et al, Erl Wood Manor, Windlesham Surrey GU20, 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

# Macrolide Antibiotics, Their Preparation And Veterinary Compositions Containing Them

This invention relates to macrolide antibiotics, in particular to compounds similar to the well-known therapeutic agent tylosin (see, for example, "Tetrahedron Letters," 2339 [1970]).

Despite tylosin's great value, there exists a constant need to develop new antibiotics, *inter alia*, in view of the possibility of emergence of resistant strains. Further, structural modification can lead to changes in the spectrum of susceptible microorganisms. Unfortunately, chemical modification of the structure of tylosin-like macrolides has proven to be extraordinarily difficult. For instance, in "J. Org. Chem.," *44* (12), 2050-2 (1979), there is an indication of the problems involved in the cleavage of the mycinosyl glycoside linkage of tylosin by chemical means. Indeed, in the overwhelming majority of cases, research workers in this area, intent on investigating novel structures, have been forced to search for new microorganisms, either naturally occurring or synthetic, hoping that their cultivation will yield related structures.

The Applicants have now discovered that compounds similar to tylosin can be prepared by the submerged aerobic fermentation of certain *Streptomyces fradiae* strains.

According to the present invention there is provided a new macrolide antibiotic having the structurel formula I—

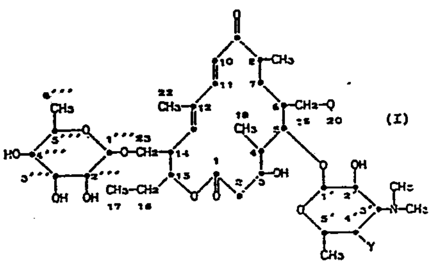

(I)

where Q is $-CHO$ or $-CH_2OH$ and Y is hydroxyl or a mycarosyloxy group; or a salt or ester thereof.

The compounds of formula I are 2‴-O-demethylmacrocin, hereinafter referred to as demethylmacrocin or DOMM and having the structural formula II—

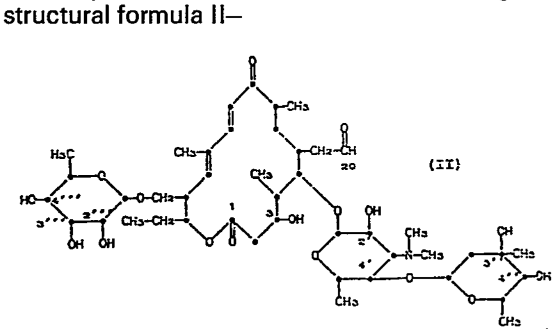

(II)

the dihydro-derivative of DOMM, i.e. 20-dihydro-2‴-O-demethylmacrocin, hereinafter referred to as dihydro-DOMM and having the structural formula III—

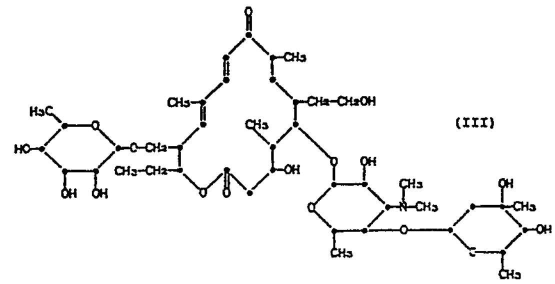

(III)

2‴-de-O-methylactenocin, hereinafter referred to as DOML, and having the structural formula IV—

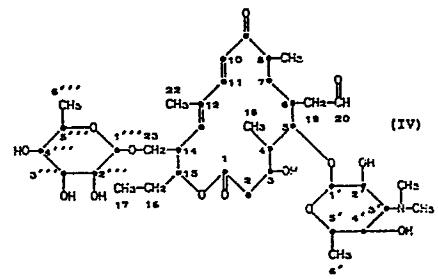

(IV)

and 20-dihydro-2‴-de-O-methylactenocin, hereinafter referred to as dihydro-DOML, and having the structural formula V—

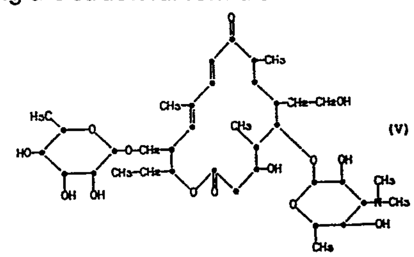

(V)

and salts and esters thereof.

The stereochemistry of the compounds of the invention is identical to that of the well-known macrolide tylosin (see, for instance, "Tetrahedron Letters," 4737 [1970]). The amino sugar is mycaminose, the left hand neutral sugar is 6-deoxy-D-allose and the neutral sugar in structures II and III is mycarose.

The hydroxyl groups of DOMM and dihydro-DOMM at the 2′, 4″, 3″, 2‴, 3‴, 4‴ and 3-positions can be esterified to form useful acyl ester derivatives. The hydroxyl groups of DOML and dihydro-DOML at the 2′, 4′, 2‴, 3‴, 4‴ and 3-positions can be esterified to form useful acyl ester derivatives. In addition, dihydro-DOMM and dihydro-DOML can be esterified on the 20-hydroxyl group. Esterification of the 2′-hydroxyl group of DOMM and dihydro-DOMM is facile. The 2′- and 4′-hydroxyl groups of DOML and dihydro-DOML are readily esterified. Typical esters are those of a monocarboxylic acid or hemi-esters of a dicarboxylic acid having from 2 to 18 carbon atoms.

DOMM, dihydro-DOMM, DOML, dihydro-DOML, and their acyl ester derivatives are basic compounds which, when combined with acids, are converted to acid addition salts. These addition salts are also part of this invention. To simplify

discussions of utility, the term DOMM compound is used and refers to DOMM, dihydro-DOMM, DOML, dihydro-DOML, a specified acyl ester derivative of these compounds, or a pharmaceutically acceptable acid addition salt of DOMM, dihydro-DOMM, DOML, dihydro-DOML, or of their acyl ester derivatives.

In a further aspect of the invention there is provided a method of preparing a novel macrolide of formula I which comprises culturing *Streptomyces fradiae* ATCC 31669 or an artificial variant or mutant thereof, under submerged aerobic conditions until a substantial level of antibiotic activity is produced, provided that if it is desired to produce a compound of formula IV or V, then the cultivation should be effected using mildly acidic conditions.

The *Streptomyces fradiae* ATCC 31669 microorganism was deposited in the American Type Culture Collection, Rockville, Maryland, U.S.A. on 21st July 1980.

DOMM is soluble in water and in most polar organic solvents, such as acetone, methanol, ethanol, chloroform, dimethylformamide, and dimethyl sulfoxide. DOMM acid addition salts are more soluble in water than is DOMM base.

DOMM can be distinguished from tylosin by paper and thin-layer chromatography. The approximate Rf and Rx values of DOMM and tylosin are summarized in Tables 1 and 2. In Table 2 Rx value is the ratio of movement expressed relative to that of tylosin, which was given a value of 1.0. Bioautography with *Bacillus subtilis* was used for detection.

*Table 1*

Thin-Layer Chromatography of DOMM[a]

| Compound | Rf Value | | |
|---|---|---|---|
| | A[b] | B | C |
| Tylosin | 0.53 | 0.53 | 0.67 |
| DOMM | 0.05 | 0.44 | 0.44 |

[a]Medium: Merck, Darmstadt—Silica Gel 60
[b]Solvent: A = ethyl acetate: diethylamine (96: 4)
B = acetone: ethanol (2: 1)
C = chloroform: methanol (3: 1)

*Table 2*

Paper Chromatography of DOMM[a]

| Compound | Rx | |
|---|---|---|
| | D[b] | E |
| Tylosin | 1.0 | 1.0 |
| DOMM | 0.17 | 0.88 |

[a]Paper: Whatman No. 1 treated with 0.75 M $KH_2PO_4$ buffer at pH 4.0 and dried
[b]Solvent: D = ethyl acetate saturated with water
E = n-butanol saturated with water

The dihydro-derivative of DOMM can be obtained by chemical reduction or by fermentation. When preparing dihydro-DOMM by chemical reduction, known procedures such as, for example, treating DOMM with an approximately stoichiometric amount of sodium borohydride in an alcoholic solvent, may be used. Dihydro-DOMM is also produced by the *Streptomyces fradiae* ATCC 31669 of this invention under controlled fermentation conditions.

This invention further relates to methods of preparing DOML and dihydro-DOML by mild acid hydrolysis of DOMM and dihydro-DOMM, respectively. Dihydro-DOML can also be prepared by (1) mild acid hydrolysis of DOMM to give DOML followed by (2) reduction of DOML to give dihydro-DOML. Mild acid hydrolysis conditions are known in the art. Appropriate aqueous solutions having a pH of about 4 or below can be used to accomplish the hydrolysis. Temperatures of about 20°C to about 100°C can be used in this method. The reaction time needed to carry out the hydrolysis varies, depending upon the pH of the reaction mixture and the temperature used. At higher pH levels the reaction rate is slower, and at higher temperatures the reaction rate is faster. At about pH 2 and at room temperature, the reaction time required is from about 6 to about 24 h. The reaction is carried out by treating either DOMM or dihydro-DOMM with a mild acid solution for a time sufficient to effect removal of the mycarosyl group to give DOML or dihydro-DOML, respectively.

Alternatively, and sometimes preferably, DOML or dihydro-DOML can be prepared by treating DOMM or dihydro-DOMM in the fermentation broth in which it is produced, using mild acidic conditions as described above for a time sufficient to convert the DOMM or dihydro-DOMM to DOML or dihydro-DOML, respectively. DOML or dihydro-DOML thus prepared can be isolated from the fermentation broth using techniques known in the art.

DOMM and dihydro-DOMM can be esterified at the 2′, 4″, 3″, 2‴, 3‴, 4‴ and 3-hydroxyl groups to give acyl ester derivatives by treatment with acylating agents using methods known in the art. DOML and dihydro-DOML can be esterified at the 2′, 4′, 2‴, 3‴, 4‴ and 3-hydroxyl groups in a similar manner. In addition, dihydro-DOMM and dihydro-DOML can be esterified at the 20-position. Esterification of the 2′-hydroxyl group of DOMM and dihydro-DOMM is facile. The 2′- and 4′-hydroxyl groups of DOML and dihydro-DOML are readily esterified. Typical acylating agents include anhydrides, halides (usually in combination with a base or other acid scavenger) and active esters of organic acids. Acylation can also be achieved by using a mixture of an organic acid and a dehydrating agent such as N,N′-dicyclohexylcarbodiimide. Acylations can also be carried out enzymatically as described by Okamoto *et al.* in U.S. Patent No. 4092473. Once formed, the acyl derivatives can be separated and purified by known techniques.

The 2′-monoester derivatives of DOMM and di-

hydro-DOMM can be prepared by selective esterification techniques generally known in the art, such as, for example, treatment of the antibiotic with a stoichiometric quantity (or a slight excess) of an acylating agent, such as an acyl anhydride, at from about 0°C to about room temperature for from about 1 to about 24 h until esterification is substantially complete. The 2′-monoester can be isolated from the reaction mixture by standard procedures such as extraction, chromatography, and crystallization.

Useful esters are those of organic acids including aliphatic, cycloaliphatic, aryl, aralkyl, heterocyclic carboxylic, sulfonic, and alkoxycarbonic acids of from 2 to 18 carbon atoms, and of inorganic acids, such as sulfuric and phosphoric acids.

Representative suitable esters include those derived from acids such as acetic, chloroacetic, propionic, butyric, isovaleric, alkoxycarbonic, stearic, cyclopropanecarboxylic, cyclohexanecarboxylic, β-cyclohexylpropionic, 1-adamantanecarboxylic, benzoic, phenylacetic, phenoxyacetic, mandelic, and 2-thienylacetic acids, and alkyl-, aryl-, and aralkylsulfonic acids, the aryl and aralkyl acids optionally bearing substituents such as halogen, nitro, lower alkoxy, and the like on the aromatic moiety. Suitable esters also include hemi-esters derived from dicarboxylic acids such as succinic, maleic, fumaric, malonic, and phthalic acids.

Pharmaceutically acceptable ester derivatives are a preferred group, such esters do not exhibit any significant toxic effects towards the warm-blooded animals to be treated. Other ester derivatives are useful, however, as intermediates.

DOMM, dihydro-DOMM, DOML, dihydro-DOML, and their specified derivatives form acid addition salts. The acid addition salts of DOMM, dihydro-DOMM, DOML, dihydro-DOML, and of their acyl derivatives are also part of this invention. Such salts are useful, for example, for separating, purifying, and crystallizing DOMM, dihydro-DOMM, DOML, dihydro-DOML, and their acyl derivatives. In addition, the salts have an improved solubility in water.

Representative suitable salts include those salts formed by standard reactions with both organic and inorganic acids such as, for example, sulfuric, hydrochloric, phosphoric, acetic, succinic, citric, lactic, maleic, fumaric, palmitic, cholic, pamoic, mucic, D-glutamic, d-camphoric, glutaric, glycolic, phthalic, tartaric, formic, lauric, stearic, salicyclic, methanesulfonic, benzenesulfonic, sorbic, picric, benzoic, cinnamic, and the like acids.

Pharmaceutically acceptable acid addition salts are an especially preferred group of salts of this invention. Pharmaceutically acceptable salts are salts which do not exhibit any significant toxic effects towards the warm-blooded animals to be treated.

The compounds of this invention will usually be administered as veterinary formulations, incorporating conventional excipients and carriers, i.e. pharmaceutically acceptable organic or inorganic carrier substances suitable for enteral, parenteral, or topical application which do not deleteriously react with the active compounds.

Suitable pharmaceutically acceptable carriers include, but are not limited to water, salt solutions, alcohols, vegetable oils, polyethylene glycols, gelatin, lactose, amylose, magnesium stearate, talc, silicic acid, paraffins, perfume oil, fatty acid monoglycerides and diglycerides, and hydroxy methylcellulose. The pharmaceutical preparations can be sterilized and, if desired, mixed with auxiliary agents, e.g., lubricants, preservatives, stabilizers, wetting agents, emulsifiers, buffers, coloring or flavoring substances which do not deleteriously react with the active compounds.

The compounds of this invention can also be employed in feed additive forms such as feed premix in conventional formulation methods.

In a further aspect of the invention there is provided a veterinary formulation comprising from 0.1 to 90% by weight of a compound of formula I, or a pharmaceutically acceptable salt or ester thereof, associated with one or more pharmaceutically acceptable carriers or excipients therefor.

This invention also provides additional methods of preparing 5-O-mycaminosyltylonolide (OMT) and 20-dihydro-5-O-mycaminosyltylonolide (dihydro-OMT). OMT and dihydro-OMT are described by Gorman *et al.* in U.S. Patent No. 3459853. In U.S. Patent No. 3459853, OMT and dihydro-OMT are prepared by removing the neutral sugars by controlled acid hydrolysis of tylosin, desmycosin, macrocin, and lactenocin and their dihydro-derivatives. This invention provides new starting materials from which OMT and dihydro-OMT can be prepared. Thus, using the method described in U.S. Patent No. 3459853, DOMM and DOML can be used to prepare OMT; and dihydro-DOMM and dihydro-DOML can be used to prepare dihydro-OMT.

DOMM and dihydro-DOMM can be prepared by culturing a strain of *Streptomyces fradiae* which produces these compounds under submerged aerobic conditions in a suitable culture medium until substantial antibiotic activity is produced. As will be appreciated by those skilled in the art, DOMM is produced first in the fermentation process. Dihydro-DOMM is produced when the fermentation is carried out for a longer time, thus permitting the DOMM present to be reduced enzymatically.

The culture medium used to grow *Streptomyces fradiae* ATCC 31669 can be any one of a number of media. For economy in production, optimal yield, and ease of product isolation, however, certain culture media are preferred. Thus, for example, preferred carbon sources in large-scale fermentation include carbohydrates such as destrin, glucose, starch, and corn meal, and oils such as soybean oil. Preferred nitrogen sources include corn meal, soybean meal, fish meal, amino acids, and the like. Among the nutrient inorganic salts which can be incorporated in the culture media are the customary soluble salts capable of yielding

iron, potassium, sodium, magnesium, calcium, ammonium, chloride, carbonate, sulfate, nitrate, and like ions.

Essential trace elements necessary for the growth and development of the organism should also be included in the culture medium. Such trace elements commonly occur as impurities in other constituents of the medium in amounts sufficient to meet the growth requirements of the organism. It may be necessary to add small amounts (i.e. 0.2 ml/l) of an antifoam agent such as polypropylene gylcol (mol.wt. about 2,000) to large-scale fermentation media if foaming becomes a problem.

For production of substantial quantities of DOMM or dihydro-DOMM, submerged aerobic fermentation in tanks is preferred. Small quantities of DOMM or dihydro-DOMM may be obtained by shake-flask culture. Because of the time lag in antibiotic production commonly associated with inoculation of large tanks with the spore form of the organism, it is preferable to use a vegetative inoculum. The vegetative inoculum is prepared by inoculating a small volume of culture medium with the spore form or mycelial fragments of the organism to obtain a fresh, actively growing culture of the organism. The vegetative inoculum is then transferred to a larger tank. The medium used for the vegetative inoculum can be the same as that used for larger fermentations, but other media can also be used.

*Streptomyces fradiae* ATCC 31669 can be grown at temperatures between about 10°C and about 37°C. Optimum antibiotic production appears to occur at temperatures of about 28°C.

As is customary in aerobic submerged culture processes, sterile air is bubbled through the culture medium. For efficient antibiotic production the percent of air saturation for tank production should be about 30% or above (at 28°C and 1 atm of pressure).

Antibiotic production can be followed during the fermentation by testing samples of the broth against organisms known to be sensitive to these antibiotics. One useful assay organism is *Staphylococcus aureus* ATCC 9144. The bioassay is conveniently performed by an automated turbidometric method. In addition, antibiotic production can be readily monitored by ultraviolet absorption of the extracted broth. High-performance liquid chromatography with UV detection can also be used to monitor production (see, for example, J.H. Kennedy in "J. Chromatographic Science," *16*, 492-495 [1978]).

Following its production under submerged aerobic fermentation conditions, DOMM or dihydro-DOMM can be recovered from the fermentation medium by methods used in the fermentation art. Recovery of DOMM or dihydro-DOMM is accomplished by an initial filtration of the fermentation broth. The filtered broth can then be further purified to give the desired antibiotic. A variety of techniques may be used in this purification. A preferred technique for purification of the filtered broth involves adjusting the broth to about pH 9; extracting the broth with a suitable solvent such as ethyl acetate, amyl acetate, or methyl isobutyl ketone; extracting the organic phase with an aqueous acidic solution; and precipitating the antibiotic by making the aqueous extract basic. Further purification involves the use of extraction, adsorption and/or crystallization techniques.

As is the case with other organisms, the characteristics of *Streptomyces fradiae* ATCC 31669 are subject to variation. For example, artificial variants and mutants of the ATCC 31669 strain may be obtained by treatment with various known physical and chemical mutagens, such as ultraviolet rays, X-rays, γ-rays, and N-methyl-N'-nitro-N-nitrosoguanidine. All natural and artificial variants, mutants, and recombinants of *Streptomyces fradiae* ATCC 31669 which retain the characteristic of production of DOMM may be used in this invention.

The DOMM compounds inhibit the growth of pathogenic bacteria, especially gram-positive bacteria and *Mycoplasma* species.

Accordingly, the novel compounds of the invention are useful in preventing, controlling, or treating microbial infections, particularly those caused by gram-positive bacteria and *Mycoplasma*.

Table 3 summarizes the minimal inhibitory concentrations (MIC), as measured by standard agar-dilution assays, at which DOMM (free base) inhibits certain bacteria.

*Table 3*

In Vitro *Activity of DOMM Free Base*

| Organism | MIC (μg/ml) |
|---|---|
| *Streptococcus pyogenes* C203 | 0.25 |
| *Streptococcus pneumoniae* Park I | 0.25 |
| *Streptococcus* sp. (Group D) 282 | 4.0 |
| *Pasteurella multocida* | 12.5 |
| *Pasteurella hemolytica* | 50.0 |
| *Mycoplasma gallisepticum* | 0.78 |
| *Mycoplasma hyopneumoniae* | 0.78 |
| *Mycoplasma hyorhinis* | 3.12 |

The DOMM compounds have shown *in vivo* antimicrobial activity against experimental bacterial infections. When two doses of test compound were administered to mice in experimental infections, the activity observed was measured as an $ED_{50}$ value (effective dose in milligrammes/kilogramme to protect 50% of the test animals: see Warren Wick *et al.*, "J. Bacteriol." *81*, 233-235 [1961]). The $ED_{50}$ values observed for DOMM are given in Table 4.

*Table 4*

*Subcutaneous $ED_{50}$ Values (mg/kg × 2) of DOMM*

| Compound | *Streptococcus pyogenes* C 203 | *Streptococcus pneumoniae* Park I | *Staphylococcus aureus* 3055 |
|---|---|---|---|
| DOMM | 1.1 | 37.5 | 3.8 |
| Bacterial Challenge (×$LD_{50}$) | 133 | 41.2 | 139 |

The following non-limitative examples are provided to more fully illustrate the invention.

*Example 1:*

A. *Shake-Flask Fermentation of DOMM*

A lyophilized pellet of *Streptomyces fradiae* ATCC 31669 was dispersed in 1 to 2 ml of sterilized water. A portion of this solution (0.5 ml) was used to inoculate a vegetative medium (150 ml) having the following composition–

| *Ingredient* | *Amount (%)* |
|---|---|
| Corn steep liquor | 1.0 |
| Yeast extract | 0.5 |
| Soybean grits | 0.5 |
| $CaCO_3$ | 0.3 |
| Soybean oil (crude) | 0.45 |
| Deionized water | 97.25 |

Alternatively, a vegetative culture of *Streptomyces fradiae* ATCC 31669 preserved, in 1 ml volumes, in liquid nitrogen was rapidly thawed and used to inoculate the vegetative medium. The inoculated vegetative medium was incubated in a 500 ml Erlenmeyer flask at 29°C for about 48 h on a closed-box shaker at 300 tr/min.

This incubated vegetative medium (0.5 ml) was used to inoculate 7 ml of a production medium having the following composition–

| *Ingredient* | *Amount (%)* |
|---|---|
| Beet molasses | 2.0 |
| Corn meal | 1.5 |
| Fish meal | 0.9 |
| Corn gluten | 0.9 |
| NaCl | 0.1 |
| $(NH_4)_2HPO_4$ | 0.04 |
| $CaCO_3$ | 0.2 |
| Soybean oil (crude) | 3.0 |
| Deionized water | 91.36 |

The inoculated fermentation medium was incubated in a 50 ml bottle at 29°C for about 6 d on a closed-box shaker at 300 tr/min.

B. *Tank Fermentation of DOMM*

In order to provide a larger volume of inoculum, 60 ml of incubated vegetative medium, prepared in a manner similar to that described in section A, was used to inoculate 40 l of a second-stage vegetative growth medium having the following composition–

| *Ingredient* | *Amount (%)* |
|---|---|
| Corn steep liquor | 1.0 |
| Soybean oil meal | 0.5 |
| Yeast extract | 0.5 |
| $CaCO_3$ | 0.3 |
| Soybean oil (crude) | 0.5 |
| Lecithin (crude) | 0.015 |
| Water | 97.185 |

The pH was adjusted to 8.5 with 50% NaOH solution.

This second-stage vegetative medium was incubated in a 68 l tank for about 40 h at 29°C, with adequate aeration and agitation.

Incubated second-stage medium (4 l) was used to inoculate 44 l of sterile production medium having the following composition–

| *Ingredient* | *Amount (%)* |
|---|---|
| Fish meal | 0.875 |
| Corn meal | 1.5 |
| Corn gluten | 0.875 |
| $CaCO_3$ | 0.2 |
| NaCl | 0.1 |
| $(NH_4)_2HPO_4$ | 0.04 |
| Beet molasses | 2.0 |
| Soybean oil (crude) | 3.0 |
| Lecithin | 0.09 |
| Water | 91.32 |

The pH was adjusted to 7.2 with 50% NaOH solution.

The inoculated production medium was allowed to ferment in a 68 l tank for about 4 d at a temperature of 28°C. The fermentation medium was aerated with sterile air to keep the dissolved oxygen level between about 30 and 50% and stirred with conventional agitators at about 250 tr/min.

*Example 2:*

*Isolation of DOMM*

Harvested whole broth (38 l), obtained as described in Example 1, was filtered with the use of a filter aid. The mycelial cake was washed with water; this water wash was then added to the filtrate.

The pH of the filtrate was adjusted to pH 9.1, using a 25% aqueous solution of sodium hydroxide. The filtrate was extracted twice with ethyl acetate (9 and 5 l). Deionized water (3500 ml) was added to the ethyl acetate extract. The pH of this solution was adjusted to about pH 4.1, using a 28% phosphoric acid solution (2 parts water/1 part concentrated phosphoric acid). After

extraction, the aqueous phase was separated. The ethyl acetate extract was extracted again with water (3500 ml) using this procedure. The two aqueous extracts were combined.

The combined aqueous extracts were adjusted to pH 8.5 with sodium hydroxide and extracted twice with chloroform (3000- and 1200-ml portions). The chloroform extracts were dried under vacuum to give about 40 g of crude DOMM base. This DOMM base was purified by crystallization from water-acetone to give about 30 g of DOMM base.

DOMM is a white solid which crystallizes from acetone-water. DOMM softens at about 135-140°C and melts completely by about 160°C. Elemental analysis of DOMM indicates that it has the following approximate percentage composition: carbon, 59.5%; hydrogen, 8%; nitrogen, 2%; oxygen, 30.5%. DOMM has an empirical formula of $C_{44}H_{73}NO_{17}$ and a molecular weight of about 888 (887, as determined by mass spectrometry).

The infrared absorption spectrum of DOMM (free base) in chloroform is shown in the accompanying drawing. Observable absorption maxima occur at the following frequencies ($cm^{-1}$): 3679 (small), 3604 (shoulder), 3486 (broad), 3012 (shoulder), 2979 (intense), 2938 (intense), 2880 (shoulder), 2821 (shoulder), 2794 (v. small), 2735 (v. small), 1723 (intense), 1678 (medium), 1627 (small), 1593 (intense), 1477 (shoulder), 1459 (medium), 1409 (medium), 1373 (medium), 1333 (shoulder), 1316 (medium), 1273 (small), 1183 (shoulder), 1161 (intense), 1114 (medium), 1080 (intense), 1048 (intense), 1013 (medium), 996 (medium), 984 (shoulder), 960 (shoulder); 924 (v. small), 902 (small), 865 (v. small), and 841 (small).

The ultraviolet absorption spectrum of DOMM in neutral ethanol exhibits an absorption maximum at 283 nm (ε 22,550).

DOMM (free base) has the following specific rotation: $[\alpha]_D^{25}$ −39.69° (c 1, $CH_3OH$).

Electrometric titration of DOMM in 66% aqueous dimethylformamide indicates the presence of a titratable group with a $pK_a$ value of about 7.14.

*Example 3:*

*Preparation of DOML*

DOMM, prepared as described in Example 2, was dissolved in a dilute hydrochloric acid solution (HCl added to water until the pH of the solution was 1.8). The resulting solution was allowed to stand for about 8 h at room temperature and was then adjusted to pH 9.0 by the addition of sodium hydroxide. This basic solution was extracted with ethyl acetate, dichloromethane or chloroform. The extract was dried under vacuum to give DOML base (softens at 115-120°C and melts at about 134-140°C).

*Example 4:*

*Préparation of Dihydro-DOMM*

DOMM (50 mg), prepared as described in Example 2, was dissolved in an aqueous isopropyl alcohol solution (approximately 40%; 25 ml). Sodium borohydride (20 mg) was dissolved in a 30% aqueous isopropyl alcohol solution (10 ml). The $NaBH_4$ solution (1 ml) was added to the solution containing the DOMM. The resulting mixture was stirred for 5 min, adjusted to pH 7.5 with phosphoric acid, and concentrated under vacuum to remove the isopropyl alcohol. Water was added to the resulting aqueous concentrate to give a volume of 25 ml; chloroform (50 ml) was then added. The pH of the aqueous phase was adjusted to 7.5. After extraction, the chloroform was separated and evaporated to dryness under vacuum to give dihydro-DOMM.

*Example 5:*

*Preparation of Dihydro-DOML*

Dihydro-DOMM, prepared as described in Example 4, was treated in the manner described in Example 3 to give dihydro-DOML.

*Example 6:*

*Alternative Preparation of DOML*

DOML was prepared from DOMM by treating the DOMM in the fermentation broth in which it was produced with mild acid as described in Example 3. Isolation of DOML was accomplished by a procedure similar to that described for DOMM in Example 2.

*Example 7:*

*Alternative Preparation of Dihydro-DOML*

DOML, obtained as described in Example 3, was reduced using the procedure described in Example 4 to give dihydro-DOML.

*Example 8:*

*2'-O-Propionyl-DOMM*

DOMM was dissolved in acetone and treated with 1.2 Eq of propionic anhydride at room temperature for about 6 h to give 2'-O-propionyl-DOMM.

*Examples 9 to 11:*

2'-O-isovaleryl-DOMM, was prepared according to the procedure of Example 8, but using isovaleric anhydride.

2'-O-benzoyl-DOMM, was prepared according to the procedure of Example 8, but using benzoic anhydride.

2'-O-(n-butyryl)-DOMM, was prepared according to the procedure of Example 8, but using n-butyric anhydride.

*Example 12:*

*2',4'-Di-O-Propionyl-DOML*

DOML was dissolved in acetone and treated with slightly more than 2 Eq of propionic anhydride at room temperature for about 6 h to give 2',4'-di-O-propionyl-DOML.

*Examples 13 to 15:*

2',4'-Di-O-isovaleryl-DOML, was prepared according to the method of Example 12, but using isovaleric anhydride.

2',4'-Di-O-benzoyl-DOML, was prepared according to the method of Example 12, but using benzoic anhydride.

2',4'-Di-O-(n-butyryl)-DOML, was prepared according to the method of Example 12, but using n-butyric anhydride.

## Claims

1. A novel macrolide of formula (I)–

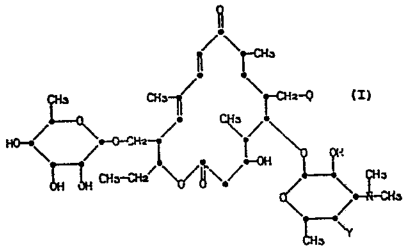
(I)

where Q is $-CHO$ or $-CH_2OH$ and Y is hydroxyl or a mycarosyloxy group; or a salt or ester thereof.

2. A novel macrolide as claimed in Claim 1, having the structural formula (II)–

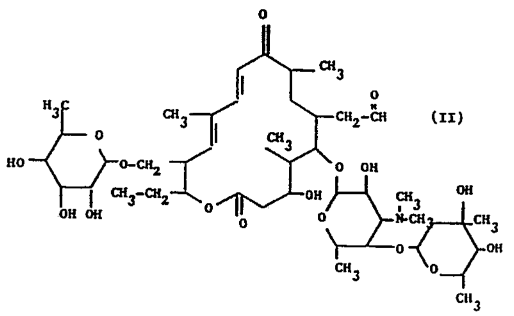
(II)

3. A novel macrolide as claimed in Claim 1, having the structural formula (III)–

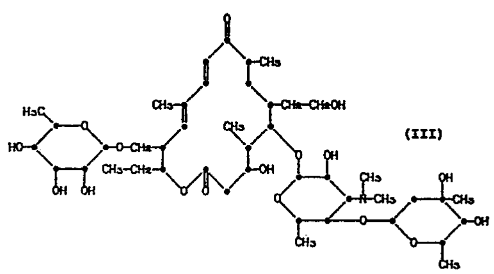
(III)

4. A novel macrolide as claimed in Claim 1, having the structural formula (IV)–

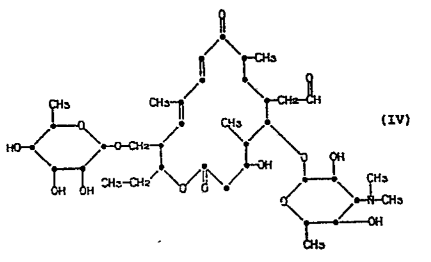
(IV)

5. A macrolide as claimed in Claim 1, having the structural formula (V)–

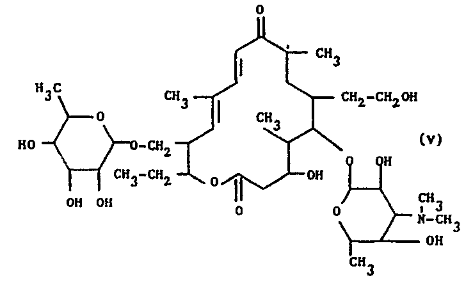
(V)

6. A method of preparing a macrolide of formula (I) as claimed in any one of Claims 1 to 5, which comprises culturing *Streptomyces fradiae* ATCC 31669, or an artificial variant or mutant thereof, under submerged aerobic conditions until a substantial level of antibiotic activity is produced, provided that if it is desired to produce a compound as claimed in Claim 4 or 5, then the cultivation should be effected using mildly acidic conditions, and isolating the macrolide.

7. A veterinary formulation comprising from 0.1 to 90% by weight of a compound of formula (I) as claimed in any one of Claims 1 to 5, or a pharmaceutically acceptable salt or ester thereof, associated with one or more pharmaceutically acceptable carriers or excipients therefor.

8. A compound of formula (I) as claimed in any one of Claims 1 to 5, or a pharmaceutically acceptable salt or ester thereof, for use in treating microbial infections in warm-blooded animals.

9. A method of preparing a compound of formula (I) as claimed in Claim 4 or 5, which comprises the mild acid hydrolysis of a compound as claimed in Claim 2 or 3 respectively.

10. A method of preparing 5-O-mycaminosyltylonolide or 20-dihydro-5-O-mycaminosyltylonolide by the controlled acid hydrolysis of a compound of formula (IV) or (V) as claimed in Claim 4 or 5.

## Revendications

1. Nouveau macrolide de formule (I):

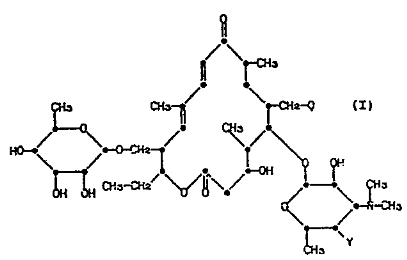
(I)

dans laquelle Q représente $-CHO$ ou $-CH_2OH$ et Y représente un groupe hydroxy ou un groupe mycarosyloxy, ou un de ses sels ou esters.

2. Nouveau macrolide suivant la revendication 1, caractérisé en ce qu'il répond à la formule structurale (II):

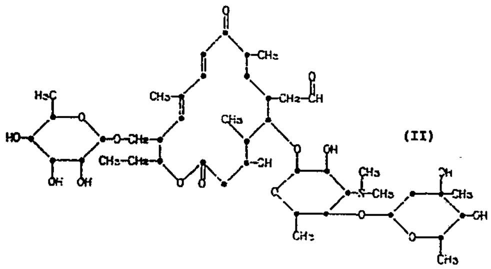

(II)

3. Nouveau macrolide suivant la revendication 1, caractérisé en ce qu'il répond à la formule structurale (III):

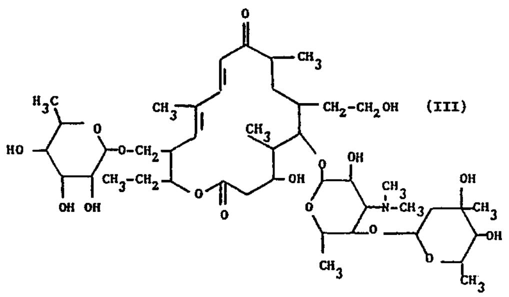

(III)

4. Nouveau macrolide suivant la revendication 1, caractérisé en ce qu'il répond à la formule structurale (IV):

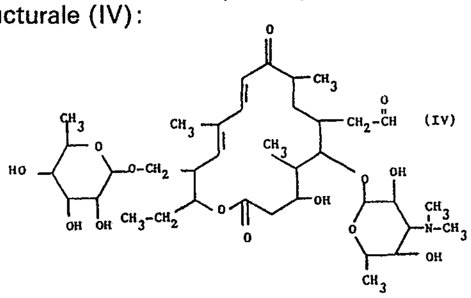

(IV)

5. Macrolide suivant la revendication 1, caractérisé en ce qu'il répond à la formule structurale (V):

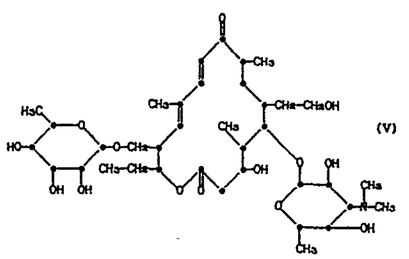

(V)

6. Procédé de préparation d'un macrolide de formule (I) suivant l'une des revendications 1 à 5, caractérisé en ce qu'il consiste à cultiver *Streptomyces fradiae* ATCC 31669, un variant ou un mutant artificiel de cette souche, dans des conditions aérobies submergées jusqu'à l'obtention d'un important degré d'activité antibiotique, avec cette restriction que, si l'on désire obtenir un composé suivant l'une des revendications 4 ou 5, on doit alors effectuer la culture en adoptant des conditions modérément acides, puis isoler le macrolide.

7. Formulation vétérinaire comprenant 0,1 à 90% en poids d'un composé de formule (I) suivant l'une des revendications 1 à 5, ou un de ses sels ou esters pharmàceutiquement acceptables, en association avec un ou plusieurs excipients ou supports pharmaceutiquement acceptables pour ce composé.

8. Composé de formule (I) suivant l'une des revendications 1 à 5, ou un de ses sels ou esters pharmaceutiquement acceptables, en vue de l'utiliser pour le traitement d'infections microbiennes chez les animaux à sang chaud.

9. Procédé de préparation d'un composé de formule (I) suivant l'une des revendications 4 ou 5, caractérisé en ce qu'il comprend l'hydrolyse acide modérée d'un composé suivant l'une des revendications 2 ou 3 respectivement.

10. Procédé de préparation du 5-O-mycaminosyltylonolide ou du 20-dihydro-5-O-mycaminosyltylonolide par hydrolyse acide contrôlée d'un composé de formule (IV) ou (V) suivant l'une des revendications 4 ou 5.

## Patentansprüche

1. Neues Makrolid der Formel (I):

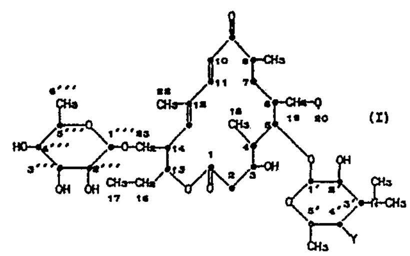

(I)

worin Q für $-CHO$ oder $-CH_2OH$ steht und Y Hydroxy oder eine Mycarosyloxygruppe ist, oder ein Salz oder Ester hiervon.

2. Neues Makrolid nach Anspruch 1, dadurch gekennzeichnet, dass es die Strukturformel (II) hat:

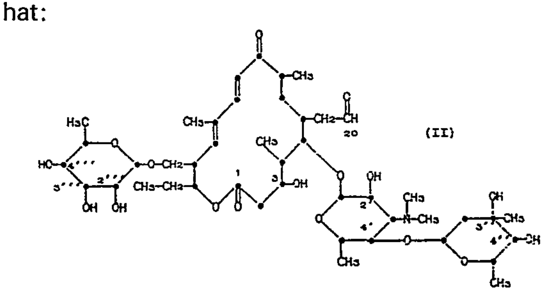

(II)

3. Neues Makrolid nach Anspruch 1, dadurch gekennzeichnet, dass es die Strukturformel (III) hat:

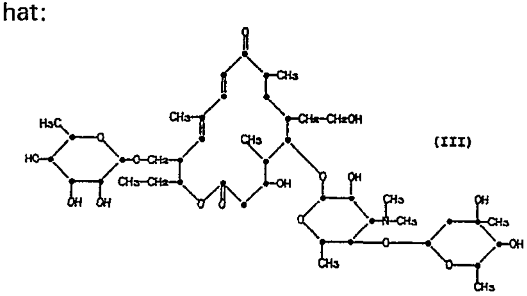

(III)

4. Neues Makrolid nach Anspruch 1, dadurch gekennzeichnet, dass es die Strukturformel (IV) hat:

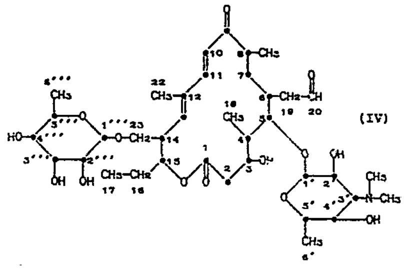

(IV)

5. Makrolid nach Anspruch 1, dadurch gekennzeichnet, dass es die Strukturformel (V) hat:

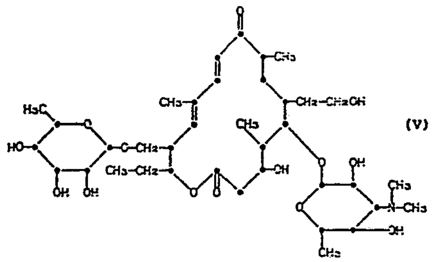

(V)

6. Verfahren zur Herstellung eines Makrolids der Formel (I) nach einem der Ansprüche 1 bis 5, dadurch gekennzeichnet, dass man *Streptomyces fradiae* ATCC 31669 oder eine künstliche Variante oder Mutante hiervon unter submersen aeroben Bedingungen bis zur Bildung einer wesentlichen Menge an antibiotischer Aktivität züchtet, wobei man, falls man eine Verbindung nach einem der Ansprüche 4 oder 5 herstellen möchte, die Züchtung dann unter Anwendung milder saurer Bedingungen durchführt, und dass man das Makrolid isoliert.

7. Veterinärformulierung aus 0,1 bis 90 Gew.-% einer Verbindung der Formel (I) nach einem der Ansprüche 1 bis 5 oder einem pharmazeutisch annehmbaren Salz oder Ester hiervon in Verbindung mit ein oder mehr pharmazeutisch annehmbaren Trägern oder Hilfsstoffen hierfür.

8. Verwendung einer Verbindung der Formel (I) nach einem der Ansprüche 1 bis 5 oder eines pharmazeutisch annehmbaren Salzes oder Esters hiervon zur Behandlung mikrobieller Infektionen bei warmblütigen Tieren.

9. Verfahren zur Herstellung einer Verbindung der Formel (I) nach einem der Ansprüche 4 oder 5, dadurch gekennzeichnet, dass man eine Verbindung gemäss einem der Ansprüche 2 oder 3 einer milden sauren Hydrolyse unterzieht.

10. Verfahren zur Herstellung von 5-O-mycaminosyltylonolid oder 20-Dihydro-5-O-mycaminosyltylonolid, dadurch gekennzeichnet, dass man eine Verbindung der Formel (IV) oder (V) gemäss einem der Ansprüche 4 oder 5 einer gesteuerten sauren Hydrolyse unterzieht.

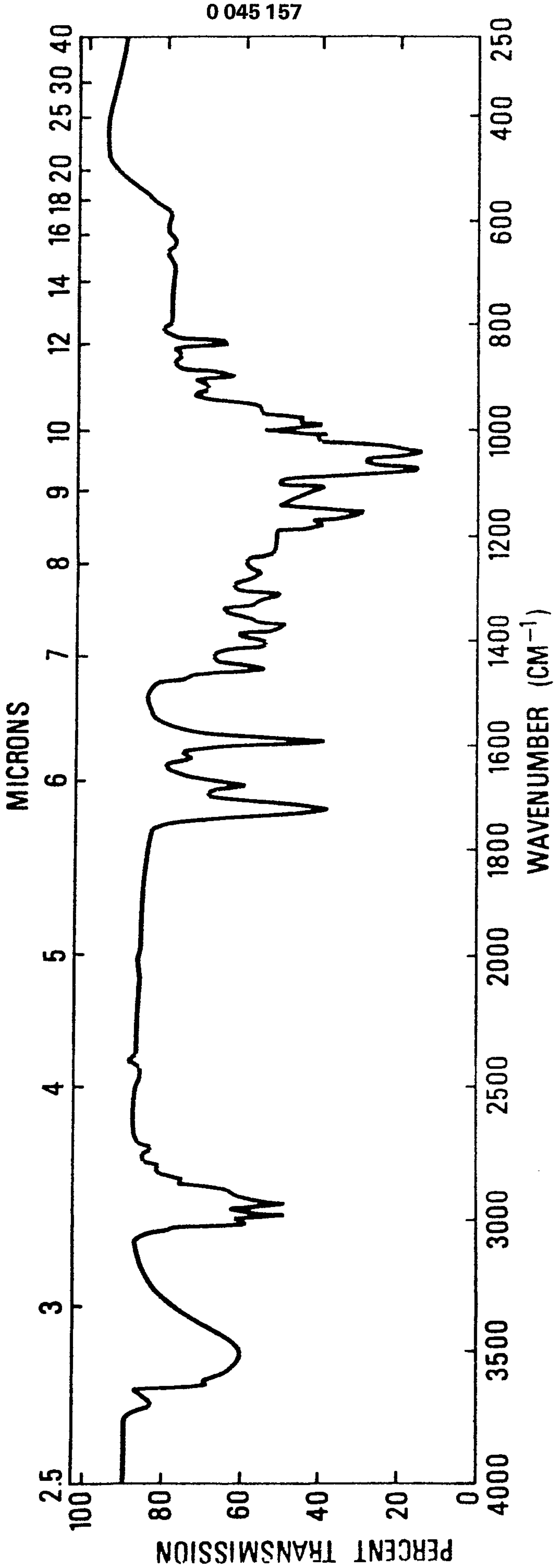
MICRONS
2.5 3 4 5 6 7 8 9 10 12 14 16 18 20 25 30 40
WAVENUMBER (CM$^{-1}$)
4000 3500 3000 2500 2000 1800 1600 1400 1200 1000 800 600 400 250
PERCENT TRANSMISSION
100 80 60 40 20 0